# EUROPEAN PATENT SPECIFICATION

(11) **EP 2 992 890 B1**
(45) Date of publication and mention of the grant of the patent: **27.05.2020**
(21) Application number: 14788988.5
(22) Date of filing: 23.04.2014
(51) Int. Cl.: A61K 36/284, A61K 36/288, A61P 29/00, A61P 1/00

(54) **PHARMACEUTICAL COMPOSITION FOR TREATMENT OF INFLAMMATORY BOWEL DISEASE**
PHARMAZEUTISCHE ZUSAMMENSETZUNG ZUR BEHANDLUNG VON CHRONISCH-ENTZÜNDLICHEN DARMERKRANKUNGEN
COMPOSITION PHARMACEUTIQUE POUR LE TRAITEMENT DES MALADIES INFLAMMATOIRES CHRONIQUE DE L' INTESTIN

(30) Priority: 24.04.2013 KR 20130045621
(43) Date of publication of application: 09.03.2016
(73) Proprietor: University-Industry Cooperation Group of Kyung Hee University, Gyeonggi-do 446-701 (KR); Ewha University-Industry Collaboration Foundation, Seoul 120-750 (KR)
(72) Inventor: KIM, Hocheol, Seoul 130-730 (KR); PARK, Juyeon, Namyangju-si Gyeonggi-do 472-719 (KR); LEE, Sung Hyun, Namyangju-si Gyeonggi-do 472-821 (KR); LEE, Donghun, Seoul 158-751 (KR); KWON, Oran, Seoul 140-763 (KR); KIM, Jiyeon, Seoul 137-761 (KR); OK, Hyangmok, Seoul 138-746 (KR)
(74) Representative: D Young & Co LLP
(86) International application number: PCT/KR2014/003541
(87) International publication number: WO 2014/175651

(56) References cited:
- KR-A- 20050 121 816
- KR-B1- 100 420 632
- US-A1- 2004 185 122
- DATABASE WPI Week 201415 Thomson Scientific, London, GB; AN 2013-V63101 XP002765286, & CN 103 285 310 A (CHINESE ACAD SCI NORTHWEST PLATEAU BIOLOGY) 11 September 2013 (2013-09-11)
- "Guide to Our Products - Singles, Combinations & Chinese", INTERNET CITATION, 14 April 2000 (2000-04-14), XP002135817, Retrieved from the Internet: URL:www.pagedepot.com/canada/tnt-herbals/p roducts.htm [retrieved on 2000-04-14]
- DATABASE MEDLINE [Online] US NATIONAL LIBRARY OF MEDICINE (NLM), BETHESDA, MD, US; May 2009 (2009-05), SHI YAN-TING ET AL: "[Screening study of the kinetogenic effects of serum containing four Chinese materia medicas on the colonic smooth muscle cells in rats].", XP002765287, Database accession no. NLM19673333 & ZHONGGUO ZHONG XI YI JIE HE ZA ZHI ZHONGGUO ZHONGXIYI JIEHE ZAZHI = CHINESE JOURNAL OF INTEGRATED TRADITIONAL AND WESTERN MEDICINE MAY 2009, vol. 29, no. 5, May 2009 (2009-05), pages 422-424, ISSN: 1003-5370
- LIU Y: "Natural five-color fruit-vegetable tea drink, e.g. for nourishing heart, strengthening spleen and stomach, and treating cancer, comprises hawthorn, radix astragali, Chrysanthemum, ginseng leaf, green tea, lucid ganoderma, and water", WPI / THOMSON,, vol. 2010, no. 6, 30 December 2009 (2009-12-30), XP002591040,
- ZHANG F ET AL: "Xiaoaisan powder for curing cancer", WPI WORLD PATENT INF, 5 December 2000 (2000-12-05), XP002951253,
- "Skin care cosmetic composition containing composite natural drug extract", DERWENT, 13 January 2000 (2000-01-13), XP002311141,
- "Medicine for curing psoriasis", DERWENT, 16 November 2001 (2001-11-16), XP002231429,
- HONG, SANG-SUN ET AL. THE JOURNAL OF KOREAN ORIENTAL INTERNAL MEDICINE. vol. 31, no. 4, 2010, pages 731 - 751, XP008181938

## Description

### [Technical Field]

The present disclosure relates to a mixed extract of *Atractylodes Rhizome white* and *Taraxacum* Herba for use in the prevention and treatment of inflammatory bowel disease, and a pharmaceutical composition for use in the prevention and treatment of inflammatory bowel disease comprising the same as an active ingredient.

### [Background Art]

*Atractylodes Rhizome white* is good for the squeamish who do not eat much, people who have malaise and a yellowish (jaundiced) skin tone, and people who have diarrhea or whose bowel movements are loose and watery. It helps water excretion when body swells and having a digestive disturbance due to water retention. It is also used for heart palpitations, cough, watery sputum among others emesis gravidarum. In addition, it is used for cold and acroesthesia accompanying gastrointestinal disorder. Pharmacologically, increase of weight, endurance and uptake activity, and promotion of immunologic function of cell in mouse were reported. Control of gastrointestinal inhibiting action and excitation, antiulcer and hepatoprotective actions, immunity accentuation, angiectasis, diuretic action, hypoglycemic action and anticancer action among others were also reported.

*Taraxacum Herba* is a whole plant of dandelion, broad-lobe dandelion, Mongolian dandelion and Korean dandelion and known to have antipyretic, antiinflammatory, diuretic and stomachic efficacies. It is used as a therapeutic agent for fever accompanying cold, sore throat, bronchitis, lymphadenitis, eye diseases, mastitis, hepatitis, cholecystitis, indigestion, urinary disorder, constipation and furuncle. It is also used as a galactagogue for folk remedy because it contains milk-like liquid in its stem and root. Substances including in the roots are fatty acid such as behenic acid, inulin, taraxerol, β-sitosterol and caffeic acid among others.

There are various types of intestinal diseases. For instance, regarding irritable bowel syndrome that is known to be a disease incurred by functional disorder, its causative organopathy cannot be detected by colonoscopy or X-ray but it is a chronic diaease accompanying reapeating abdominal pain after eating or getting stressed, unpleasant digestive symptoms such as abdominal distention, and dyschezia such as diarrhea or constipation. Generally, spasmolytic, alleviator and some tranquilizer are used for drug treatment.

Meanwhile, inflammatory bowel disease (IBD) is a disease including ulcerative colitis, Crohn's disease and Behcet disease among others and its critical etiology is known to be an activation of inflammatory cells. It requires treatment such as inhibition on activation of inflammatory cells by using antiinflammatory agents or immunosuppressive agents.

That is, adequate treatment method should be selected and applied to each types of intestinal disease while considering factors such as specific etiology among others. Especially, preparation of plan for inhibition and alleviation to inflammation related symptoms is the most significant element in regard of treating inflammatory bowel disease.

Continual or inadequate activation in bowel immune system plays an important role in pathophysiology of chronic mucosal inflammation and especially, it incurs mucosal destruction and ulcer consequently due to infiltration of neutrophil, macrophage, lymphocyte and mastocyte.

Infiltrated and activated neutrophil becomes a major cause of reactive oxygen/nitrogen species. These reactive species induce cellular oxidative stress due to cross-linking protein, lipid and hexane and incur epithelial dysfunction and epithelial damage as cytotoxic substances.

Various inflammatory cytokine are secreted from intestinal mucous membrane when having inflammatory bowel disease. TNF-α is shown high in intestine lumen and intestinal epithelial cell of ulcerative colitis patients, and TNF-α is known to play an important role as a cause of ulcerative colitis according to recent studies. Infliximab (i.e. Anti-TNF-α antibody) is known to be effective in treating furuncle as well as Crohn's disease which could not be treated heretofore. However, this treatment is expensive and incurs side effects such as fluid responsiveness or complications of communicable diseases among others in some patients.

Currently, drugs of 5-aminosalicylic acid (5-ASA) system which inhibit generation of prostaglandins such as sulfasalazine, immunosuppressant drugs of steroids among others are used for treating inflammatory bowel diseases.

Sulfasalazine tends to incur side or adverse effects such as fullness, headache, rash, liver diseases, leukopenia, agranulocytosis and male sterility among others. Also, it is unclear whether sulfasalazine has enough recurrence inhibiting effect for patients whose affected area of bowel is excised or who are showing an improvement.

Immunosuppressant drugs of steroids are adrenal cortex steroids and recognized of its short-term effect, but it cannot improve long-term prognosis and have limited use that it should only be applied for acute diseases apropos of side effects such as induced inflammatory diseases, secondary adrenocortical insufficiency, peptic ulcer, diabetes, insanity and steroidal kidney diseases among others.

Meanwhile, natural extracts and preparations using natural extracts have been used for relieving and treating various diseases including cough, cold, parasite infection and inflammation. More than 60% of anti-cancer agents that currently released in the market and more than 75% of infectious diseases agents are originated in natural products because there is wide variety of natural product providing highly specific physiological activation.

KR 100420632 relates to a composition for treating Behcet's disease comprising at least one or more type of extract selected from a group consisiting of *Atractylodis* Rhizoma alba or Taraxacum platycarpum amongst others.

However, no credible treating methods for inflammatory bowel diseases exist so far and accordingly development of effective therapeutic agents for these diseases is required. Therefore, helpful natural extracts on inflammatory bowel diseases are necessary.

### [Summary of Invention]

### [Technical Problem]

It is an object of the present disclosure to provide a mixed extract of *Atractylodes Rhizome white* and *Taraxacum Herba* for use in the prevention and treatment of inflammatory bowel disease.

Also described herein is a pharmaceutical composition for use in preventing or treating inflammatory bowel disease comprising the mixed extract of *Atractylodes Rhizome white* and *Taraxacum Herba* as an active ingredient.

Also described herein is a food composition for improving inflammatory bowel disease comprising the mixed extract of *Atractylodes Rhizome white* and *Taraxacum Herba* as an active ingredient.

### [Solution to Problem]

The present disclosure provides a mixed extract of *Atractylodes Rhizome white* and *Taraxacum Herba* for use in the prevention and treatment of inflammatory bowel disease.

*Atractylodes Rhizome white* of the present disclosure is directed to a rhizome of plant or periderm-removed plant including *Atractylodes japonica, Atractylodes macrocephala* and *Atractylodes ovate* among others. Preferably, *Atractylodes Rhizome white* is originated from *Atractylodes macrocephala.* It is generally available to purchase from markets in Korea, China and Japan among others.

*Taraxacum Herba* of the present disclosure is directed to an entirety of dandelion, broad-lobe dandelion, Mongolian dandelion and Korean dandelion, and includes plants originated from edible genus Taraxacum such as *Taraxacum platycarpum, Taraxacum officinale, Taraxacum mongolicum, Taraxacum coreanum, Taraxacum albidum, Taraxacum glabrisquamum, Taraxacum pseudoalbidum* and *Taraxacum taquetii* among others. These are available to purchase in domestic and foreign markets such as Korea, China and Japan, and these are sold as mixed ingredients because generally the difference of activity among genus Taraxacum are little.

The mixed extract of *Atractylodes Rhizome white* and *Taraxacum Herba* of the present disclosure can be obtained by extracting mixture of *Atractylodes Rhizome white* and *Taraxacum Herba* or by mixing each extract of *Atractylodes Rhizome white* and *Taraxacum Herba.* The mixed extract of *Atractylodes Rhizome white* and *Taraxacum Herba* of the present disclosure can be a form of concentrated liquid or lyophilized powder.

The mixed extract of *Atractylodes Rhizome white* and *Taraxacum Herba* of the present disclosure can be extracted by a solvent selected from a group consisting of distilled water, C₁₋₄ alcohol, ethyl acetate, glycerin, ethylene glycol, propylene glycol, butylene glycol, and its mixed solvent (e.g. hydrous alcohol, hydrous glycerin, hydrous ethylene glycol, hydrous propylene glycol, hydrous butylene glycol) having 1-30 times of volume based on dry weight of *Atractylodes Rhizome white* or *Taraxacum Herba;* preferably by a solvent selected from a group consisting of water, C₁₋₄ anhydrous or C₁₋₄ hydrous alcohol; and more preferably by water. The mixed extract of *Atractylodes Rhizome white* and *Taraxacum Herba* can be extracted by hot-water extraction under 30-120 °C for 1-48 hours and preferably by hot-water extraction under 80°C-100°C for 2-4 hours. It can be repeatedly extracted for 1-5 times. Additionally, it can be yielded by concentrating extraction solvents by using a vacuum evaporator.

Also, the mixed extract of *Atractylodes Rhizome white* and *Taraxacum Herba* of the present disclosure can be yielded by mixing *Atractylodes Rhizome white* and *Taraxacum Herba* then preparing by following the said method.

Furthermore, the mixed extract of *Atractylodes Rhizome white* and *Taraxacum Herba* of the present disclosure can be prepared by using the known ultrasonic-assisted extraction with the said extraction solvents.

Mixed weight ratio of the mixed extract of *Atractylodes Rhizome white* and *Taraxacum Herba* of the present disclosure can be varied but it is preferable to be mixed in the ratio of 1:2-2:1 based on the dry weight, and more preferably in the ratio of 1:1 based on the dry weight.

The present disclosure also provides a pharmaceutical composition for use in preventing or treating inflammatory bowel disease comprising the mixed extract of *Atractylodes Rhizome white* and *Taraxacum Herba* as an active ingredient.

Any *Atractylodes Rhizome white* and *Taraxacum Herba* originated from the said kinds can be used but preferably, *Atractylodes Rhizome white* originated from Atractylodes macrocephala has more remarkable effects in preventing and treating inflammatory bowel disease when used as the mixed extract with *Taraxacum Herba.*

The mixed extract of *Atractylodes Rhizome white* and *Taraxacum Herba* of the present disclosure has effects in preventing and treating inflammatory bowel disease through significant inhibition on inflammatory response of inflammatory bowel disease.

Mixed use of *Atractylodes Rhizome white* and *Taraxacum Herba* has superior effect in preventing and treating inflammatory bowel disease compared to separated use of each *Atractylodes Rhizome white* extract or *Taraxacum Herba* extract. That is, the mixed extract of *Atractylodes Rhizome white* and *Taraxacum Herba* has superior effect in preventing and treating inflammatory bowel disease compared to each extract of *Atractylodes Rhizome white* or *Taraxacum Herba.*

It is preferable for the mixed extract of *Atractylodes Rhizome white* and *Taraxacum Herba* of the present disclosure to be included 0.1-80.0 wt% based on total weight of composition in form of powder or liquid, and more preferably to be included 20.0-40.0 wt%, but not limited to these.

The composition of the present disclosure can additionally include pharmaceutically acceptable additives such as starch, gelatinized starch, microcrystalline cellulose, milk sugar, povidone, colloidal silicon dioxide, calcium hydrogen phosphate, lactose, mannitol, malt, gum arabic, pre-gelatinized starch, corn starch, powdered cellulose, hydroxypropyl cellulose, opadry, sodium glycolate starch, carnauba wax, synthetic aluminum silicate, stearic acid, magnesium stearate, aluminium stearate, calcium stearate, sucrose, dextrose, sorbitol and talc among others.

It is preferable for the pharmaceutically acceptable additives of the present disclosure to be included 0.1-90.0 wt% based on 100.0 wt% of the mixed extract of *Atractylodes Rhizome white* and *Taraxacum Herba,* and more preferably to be included 10.0-50.0 wt%, but not limited to these.

That is, the composition ot the present disclosure can be administered by various formulations as orally and parenterally when conducting actual clinical administration and preparations can be prepared by using common diluents or excipients such as fillers, extenders, binders, humectants, disintegrants and surfactants among others. Solid preparations for oral administration include tablets, pills, powders, granules and capsules among others, and these solid preparations can be prepared by mixing one or more excipients (e.g. starch, calciumcarbonate, sucrose, lactose or gelatin among others) with the mixed extract of *Atractylodes Rhizome white* and *Taraxacum Herba.* Additionally, lubricants such as magnesium stearate talc among others can be used other than simple excipients. Liquid preparations for oral administration include suspensions, liquid for internal use, emulsions and syrups among others, and various excipients such as humectants, sweetening agents, aromatic agents and preservatives among others can also be included as well as water and liquid paraffin which are simple diluents. Preparations for parenteral administration can include sterile aqueous solutions, nonaqueous solvents, suspensions, emulsions, lyophilizer and suppositories. Propylene glycol, polyethylene glycol, vegetable oils such as olive oil and injectable esters such as ethyl oleate among others can be used as the nonaqueous solvents and suspensions. Witepsol, macrogol, tween 61, cacao butter, laurinum and glycerogelatin among others can be used as base compound of suppositories.

The composition of the present disclosure can be administered orally or parenterally depending on methods. It is preferable for injection selected from dermatological or intraperitoneal injections, intrarectal injection, hypodermic injection, venous injection, intramuscular injection or intrathoracic injection when applying parenteral administration.

Dosage of the composition of the present disclosure varies upon patient's weight, age, sex, health, diet, administration time, administration method, excretion rate and severity of disease, and it is preferable for daily dosage to be 0.0001-200.0 mg/kg based on quantity of the mixed extract of *Atractylodes Rhizome white* and *Taraxacum Herba.* Administration can be once a day or divided into several times per a day. However, the said dosage explained hereinbefore does not limit scope of the present disclosure.

Furthermore, the present disclosure provides a food composition for improving inflammatory bowel disease comprising the mixed extract of *Atractylodes Rhizome white* and *Taraxacum Herba* as an active ingredient.

*Atractylodes Rhizome white* and *Taraxacum Herba* can be added to a food composition as they are or used with other foods or food ingredients, and can be used properly in the usual manner.

There is no particular limitation on the foods. The foods whereto the mixed extract of *Atractylodes Rhizome white* and *Taraxacum Herba* can be added include chocolates, candies, snacks, noodles, gums, soups, beverages, tees, drinks, alcoholic drinks and vitamin complexes among others. The foods include all kinds of health food in the conventional sense.

Other than the above, the mixed extract of *Atractylodes Rhizome white* and *Taraxacum Herba* of the present disclosure can contain various nutritional supplements, vitamins, electrolytes, favoring agents, coloring agents, pectic acid and its salt, alginic acid and its salt, organic acid, protective colloid thickener, pH modifier, stabilizer, preservative, glycerin, alcohol and carbonator for carbonated beverage among others. Moreover, the mixed extract of *Atractylodes Rhizome white* and *Taraxacum Herba* of the present disclosure can contain pulps for preparing natural fruit juices, fruit juices and vegetable drinks. These ingredients can be used solely or mixedly.

The use of the present disclosure for treating inflammatory bowel disease is not only related to the very disease itself before expression of antecedent symptoms but also includes inhibiting or avoiding the symptoms. In managing diseases, preventive or therapeutical amount of specific active ingredient would vary depending on nature and severity of the diseases and routes of administrating the active ingredients. Amount and frequency of administration would vary depending on age, weight and response of each patient. Adequate dosage can be easily selected by those skilled in the art who obviously consider these factors. Additionally, the mixed extract according to the present disclosure for use in preventing or treating inflammatory bowel disease can further include additional active preparations that are helpful in treating diseases and the additional active preparations can exhibit synergistic effect or adjuvant effect when using with the mixed extract of *Atractylodes Rhizome white* and *Taraxacum Herba.*

### [Advantageous Effect]

The mixed extract of *Atractylodes Rhizome white* and *Taraxacum Herba* of the present disclosure is effective for use in preventing or treating inflammatory bowel disease through its inflammatory inhibitory effects on inflammatory bowel disease.

### [Brief Description of Drawings]

Figure 1 shows a result of a measurement on weight variation upon administrating the mixed extract of *Atractylodes Rhizome white* and *Taraxacum Herba* to an ulcerative colitis animal model.
Figure 2 shows a result of a measurement on length variation of intestine upon administrating the mixed extract of *Atractylodes Rhizome white* and *Taraxacum Herba* to the ulcerative colitis animal model.
Figure 3 shows a result of a confirmation on histological change upon administrating the mixed extract of *Atractylodes Rhizome white* and *Taraxacum Herba* to the ulcerative colitis animal model.
Figure 4 shows a result of a measurement on disease activity index in the ulcerative colitis animal model upon administrating the mixed extract of *Atractylodes Rhizome white* and Taraxacum *Herba.*
Figure 5 shows a result of a measurement on activity of myeloperoxidase in the ulcerative colitis animal model upon administrating the mixed extract of *Atractylodes Rhizome white* and *Taraxacum Herba.*

### [Description of Embodiments]

The present disclosure will be described more fully hereinafter with reference to the accompanying examples. However, the following examples are intended to illustrate the present invention, and the present invention is not limited by the following examples.

### Example 1: Preparation of the mixed extract of Atractylodes Rhizome white and Taraxacum Herba

### <1-1> Preparation of the mixed extract of Atractylodes Rhizome white and Taraxacum Herba

10 times weight of water was added to 50g of *Atractylodes Rhizome white* (i.e. originated from Atractylodes macrocephala, country of origin: China, purchased from Daewoo herb wholesaler) and it was repeatedly extracted 2 times under 100 °C in 3 hours. 19.4g of powdered *Atractylodes Rhizome white* extract was prepared by concentrating filtrates of the above extraction under reduced pressure (i.e. 40 brix) and lyophilizing the same (yield: 38.8%).

*Taraxacum Herba* (country of origin: China, purchased from Daewoo herb wholesaler) extract was prepared in the same manner as *Atractylodes Rhizome white.* 8.48g of *Taraxacum Herba* extract was prepared by using 50g of *Taraxacum Herba* (yield: 17.0 %).

Mixed extract was prepared by mixing the prepared *Atractylodes Rhizome white* extract and the *Taraxacum Herba* extract to have constitutions and weight ratio as stated in Table 1 below.

**[Table 1]**

| | | *Atractylodes Rhizome white* | *Taraxacum Herba* |
|---|---|---|---|
| Example | Weight (mg) | 50 | 50 |
| | Weight Ratio | 1 | 1 |

### <1-2> Preparation of the Atractylodes Rhizome white extract (Comparative Example 1)

10 times weight of water was added to 50g of *Atractylodes Rhizome white* (i.e. originated from Atractylodes macrocephala, country of origin: China) and it was repeatedly extracted 2 times under 100 °C in 3 hours. 19.4g of powdered *Atractylodes Rhizome* white extract was prepared by concentrating filtrates of the above extraction under reduced pressure (i.e. 40 brix) and lyophilizing the same (yield: 38.8%).

### <1-3> Preparation of the Taraxacum Herba extract (Comparative Example 2)

10 times weight of water was added to 50g of *Taraxacum Herba* (country of origin: China) and repeatedly extracted 2 times under 100 °C in 3 hours. 8.48g of powdered *Taraxacum Herba* extract was prepared by concentrating filtrates of the above extraction under reduced pressure (i.e. 40 brix) and lyophilizing the same (yield: 17.0%).

### Example 2: Confirmation on treating effect of the mixed extract of Atractylodes Rhizome white and Taraxacum Herba for inflammatory bowel disease

Ulcerative colitis was induced by voluntarily drinking aqueous solution of 5% Dextran Sulfate Sodium (DSS) for 7 days in 8-week old masculine mice (C57BL/6, Samtako BIO Korea Inc.).

According to the DSS administration, treatment effect on inflammatory bowel disease upon administration of the mixed extract of *Atractylodes Rhizome white* and *Taraxacum Herba* after induction of ulcerative colitis was confirmed. Experimental groups and administered extracts for each experimental group were set as stated in Table 2 below.

**[Table 2]**

| Experimental Groups | Administered Extracts |
|---|---|
| Normal Group | Distilled Water |
| Control Group | DSS + Distilled Water |
| Comparative Example 1 (*Atractylodes Rhizome white* Extract Administered Group) | DSS + < Comparative Example 1> 100mg/kg of the *Atractylodes Rhizome white* Extract |
| Comparative Example 2 (*Taraxacum Herba* Extract Administered Group) | DSS + < Comparative Example 2> 100mg/kg of the *Taraxacum Herba* Extract |
| Mixed Extract of *Atractylodes Rhizome white* and *Taraxacum Herba* Administered Group (100mg/kg) | DSS + <Example 1> 100mg/kg of the Mixed Extract of *Atractylodes Rhizome white* and *Taraxacum Herba* |
| Mixed Extract of *Atractylodes Rhizome white* and *Taraxacum Herba* Administered Group (30mg/kg) | DSS + <Example 1> 30mg/kg of the Mixed Extract of *Atractylodes Rhizome white* and *Taraxacum Herba* |

Two times of oral drug administration per a day were conducted for 7 days, and environment was controlled to have 22±2°C of temperature, 50 ± 10% of relative humidity and an illumination cycle (i.e. 07:00 lights on/19:00 lights off). Weights of the mice were measured once a day during the test period and lengths of intestine of every experimental group were measured at the last day of the test by sacrificing all experimental groups. Measured lengths of intestine were analyzed by converting the lengths (cm) into percentages (%).

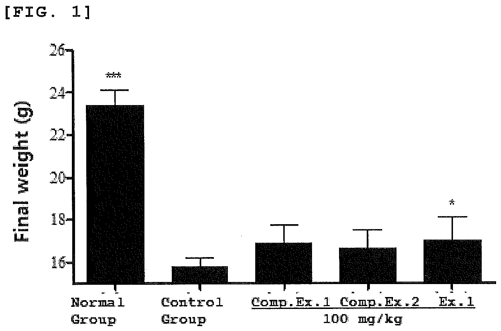

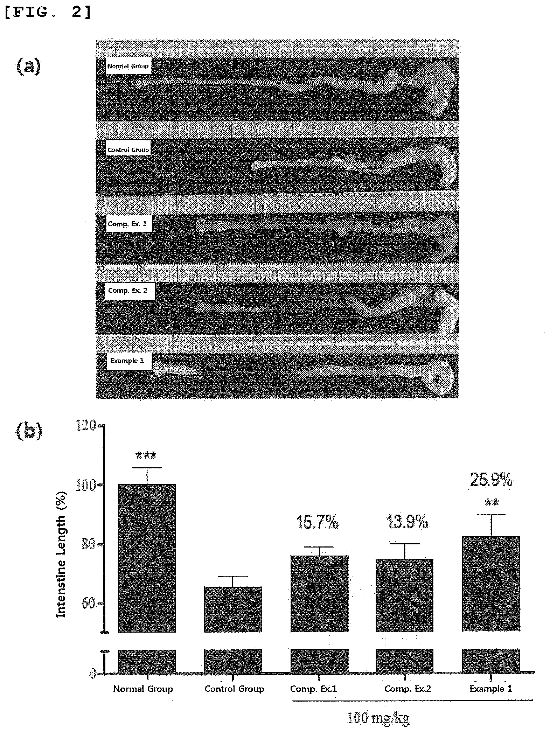

Results are shown in Figures 1 and 2.

Figure 1 shows effect of weight recovery upon administration of the mixed extract of *Atractylodes Rhizome white* and *Taraxacum Herba.* As a result of administrating the samples for 7 days on each administration group, weights (g) of the groups wherein 100mg/kg of the *Atractylodes Rhizome white* extract and 100mg/kg of the *Taraxacum Herba* extract were administered were increased as 7.1% and 5.6% respectively compared to the control group but it is not statistically significant. However, weight (g) of the group wherein 100mg/kg of the mixed extract of *Atractylodes Rhizome white* and *Taraxacum Herba* was administered was significantly increased as 7.9% compared to that of the control group (23.4±0.74g vs. 17.0±1.08g, P<0.1) (mean ± SD). This result shows that effect of the mixed extract of *Atractylodes Rhizome white* and *Taraxacum Herba* is more remarkable than that of separate use of the *Atractylodes Rhizome white* extract and the *Taraxacum Herba* extract.

Figure 2 shows effects of treating inflammation upon administration of the mixed extract of *Atractylodes Rhizome white* and *Taraxacum Herba.* The treating effect of the mixed extract of *Atractylodes Rhizome white* and *Taraxacum Herba* could be confirmed through length variation of intestine because length of intestine was shortening due to inflammation response which was caused by DSS administration.

It was confirmed that length of intestine (%) decreased markedly in the DSS-induced control group compared to the normal group due to tissue damage, and recovery effect upon administration of the mixed extract of *Atractylodes Rhizome white* and *Taraxacum Herba* was also confirmed with the naked eye. Compared to the control group, length variation of actual intestine converted into a percentage was also significantly increased by 25.9% (65.5±3.6% vs. 82.4±7.4%, P<0.01)in the mixed extract of *Atractylodes Rhizome white* and *Taraxacum Herba* administered group and it showed far superior effect to separate use of the *Atractylodes Rhizome white* extract or *Taraxacum Herba* extract. The result shows that using the mixed extract of *Atractylodes Rhizome white* and *Taraxacum Herba* has more remarkable effect than using the *Atractylodes Rhizome white* extract or the *Taraxacum Herba* extract separately in treating inflammatory bowel disease.

### Example 3: Confirmation on histological change upon administration of the mixed extract of Atractylodes Rhizome white and Taraxacum Herba

The change of intestinal tissue was confirmed by using the intestine obtained in Example 2.

Experimental animals were sacrificed; colon tissues were removed; they were rinsed with PBS and fixed with buffered formalin. And then, they were embedded in paraffin through ethanol dehydration and sliced. They were stained by Hematoxylin-Eosin (H&E) staining and observed through a microscope.

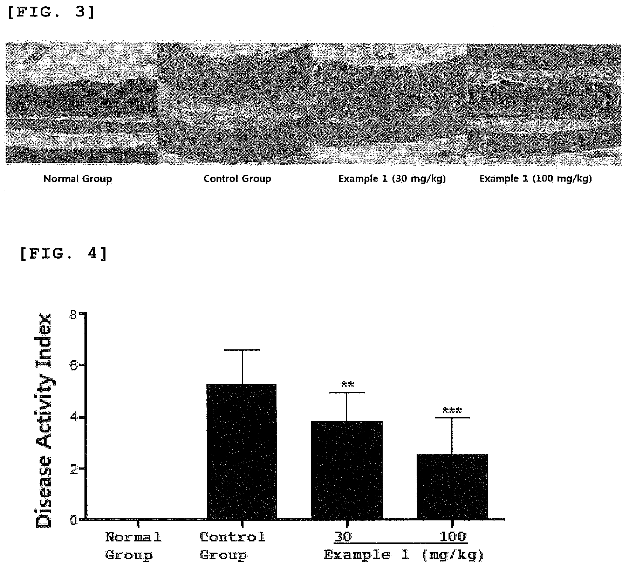

Result is shown in Figure 3. As shown in Figure 3, intestine tissue was not clear due to destructed cryptal area and goblet cell in various region of mucosal epithelial layer. However, intestine tissue structure of the mixed extract of *Atractylodes Rhizome white* and *Taraxacum Herba* (30 mg/kg, 100 mg/kg) administered group in Example 1 was clear compared to the control group, and it was confirmed that this group showed a tissue structure which was similar to that of the normal group.

### Example 4: Confirmation on disease activity index upon administration of the mixed extract of Atractylodes Rhizome white and Taraxacum Herba

Disease activity index of ulcerative colitis model in Example 2 was analyzed.

Behavior, degree of piloerection, diarrhea and hemorrhage, and colon edema after intestine removal were measured pursuant to criteria set forth in Table 3 below and analyzed in regard of the disease activity index. Behavior, degree of piloerection and diarrhea and hemorrhage were measured daily during 7 days of sample administration and colon edema of intestine was measured right after the sacrifice.

**[Table 3] Disease activity index**

| **1. Behavior Test** | **Score** |
|---|---|
| Motion(---) with hunched(+++) | 4 |
| Motion(--) + hunched(++) | 3 |
| Motion(-) + hunched(+) | 2 |
| Motion(+) + hunched(-) | 1 |
| Motion(+) | 0 |

| **2. Piloerection + pilo color** | |
|---|---|
| Yellow(brown)(+++) + piloerection(+++) | 4 |
| Yellow(++) + piloerection(++) | 3 |
| Yellow(light)(+) + piloerection (+) | 2 |
| White yellow + piloerection(-) | 1 |
| White(0) + No piloerection(-) | 0 |

| 3. Cleaning of anus | |
|---|---|
| Watery Diarrhea(+++) + anal prolapsed +bleeding(+++) | 4 |
| Loose feces(++) + bleeding(++) | 3 |
| Slightly loose feces(+) + bleeding(+) | 2 |
| Bleeding(-) | 1 |
| Health state | 0 |

| 4. Gross anatomy | |
|---|---|
| Edema(+++, 0.35 mm) | 4 |
| Edema(++, 0.3∼0.35 mm) | 3 |
| Edema(+, 0.25∼0.30 mm) | 2 |
| Edema(+-, 0.2∼0.25mm) | 1 |
| Normal(0.1∼0.2mm in colon thickness) | 0 |

The result was illustrated in Figure 4. As illustrated in Figure 4, it was confirmed that disease activity index was dose-dependently decreased. In addition, disease activity index was dose-dependently decrease as significantly by 3.80±0.36 or 2.53±0.36 in the mixed extract of *Atractylodes Rhizome white* extract and the *Taraxacum Herba* extract administrated group of example 1 (30 mg/kg or 100 mg/kg) compared to the control group.

### Example 5: Confirmation on activity variation of myeloperoxidase upon administration of the mixed extract of Atractylodes Rhizome white and Taraxacum Herba

To confirm deposition degree of polymorphonuclear leukocyte, activity variation of myeloperoxidase was confirmed by using the removed intestine tissue in Example 2.

To measure myeloperoxidase activity, colon tissue was homogenated by suspending colon tissues in 50 mM potassium phosphate buffer (pH 6.5) containing hexa-decyl-trimethyl-ammonium bromide (HETAB); 3 times of freezing-thawing and ultrasonication was repeatedly conducted. And then, supernatants was obtained by conducting centrifugation for 30 minutes under 20,000 rpm and 4°C; and the same was used as an enzyme liquid. 10 µl of the prepared enzyme liquid was reacted for 3 minutes by putting the same in O-dianisidine and 190 µl of 50 mM potassium phosphate buffer (pH 6.0) containing hydrogen peroxide (H₂O₂), and absorbance was measured by using ELISA microplate reader (SpectraMax Plus384, Molecular Devices, Sunnyvale, USA) under 470nm. Result was denoted in unit of myeloperoxidase (MPO) per 1 mg of tissue, and 1 unit is defined as an activity that can reduce 1 µmol of hydrogen peroxide to water.

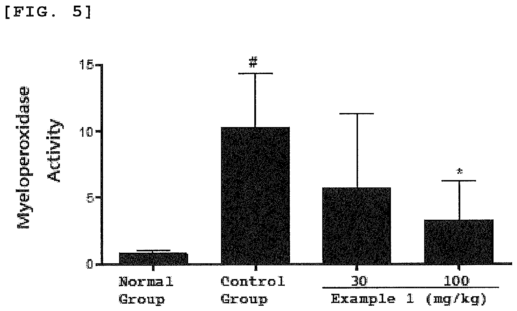

Result is illustrated in Figure 5. As illustrated in Figure 5, myeloperoxidase activity was dose-dependently decrease as significantly by 5.67±5.66 or 3.26±3.0 respectively in the mixed extract of *Atractylodes Rhizome white* and *Taraxacum Herba* (30 mg/kg, 100 mg/kg) administered group in Example 1 compared to control group.

## Claims

1. A mixed extract of *Atractylodes Rhizome white* and *Taraxacum Herba* for use in the prevention and treatment of inflammatory bowel disease.

2. The mixed extract of *Atractylodes Rhizome white* and *Taraxacum Herba* for use according to claim 1, wherein:
the *Atractylodes Rhizome white* is originated from one or more selected from a group consisting of *Atractylodes japonica, Atractylodes macrocephala* or *Atractylodes ovate*; and
the *Taraxacum Herba* is originated from one or more selected from a group consisting of *Taraxacum platycarpum, Taraxacum officinale, Taraxacum mongolicum, Taraxacum coreanum, Taraxacum albidum, Taraxacum glabrisquamum, Taraxacum pseudoalbidum* or *Taraxacum taquetii.*

3. The mixed extract of *Atractylodes Rhizome white* and *Taraxacum Herba* for use according to claim 2, wherein the *Atractylodes Rhizome white* is originated from the Atractylodes macrocephala.

4. The mixed extract of *Atractylodes Rhizome white* and *Taraxacum Herba* for use according to claim 3, wherein the mixed extract of *Atractylodes Rhizome white* and *Taraxacum Herba* is extracted with one or more solvent selected from a group consisting of water, C₁₋₄ alcohol or mixtures thereof.

5. The mixed extract of *Atractylodes Rhizome white* and *Taraxacum Herba* for use according to claim 4, wherein a mixed dry weight ratio of the mixed extract of *Atractylodes Rhizome white* and *Taraxacum Herba* is 1:2-2:1.

## Patentansprüche

1. Gemischter Extrakt von *Atractylodes Rhizome white* und *Taraxacum Herba* zur Verwendung bei der Prävention und Behandlung von entzündlicher Darmkrankheit.

2. Gemischter Extrakt von *Atractylodes Rhizome white* und *Taraxacum Herba* zur Verwendung nach Anspruch 1, wobei:
das *Atractylodes Rhizome white* aus einem oder mehreren ausgewählt aus einer Gruppe bestehend aus *Atractylodes japonica, Atractylodes macrocephala* und *Atractylodes ovate* stammt und
das *Taraxacum Herba* aus einem oder mehreren ausgewählt aus einer Gruppe bestehend aus *Taraxacum platycarpum Taraxacum officinale, Taraxacum mongolicum, Taraxacum coreanum, Taraxacum albidum, Taraxacum glabrisquamum, Taraxacum pseudoalbidum* oder *Taraxacum taquettii* stammt.

3. Gemischter Extrakt von *Atractylodes Rhizome white* und *Taraxacum Herba* zur Verwendung nach Anspruch 2, wobei das *Atractylodes Rhizome white* aus *Atractylodes macrocephala* stammt.

4. Gemischter Extrakt von *Atractylodes Rhizome white* und *Taraxacum Herba* zur Verwendung nach Anspruch 3, wobei der gemischte Extrakt von *Atractylodes Rhizome white* und *Taraxacum Herba* mit einem oder mehreren Lösungsmitteln ausgewählt aus einer aus Wasser, C₁₋₄-Alkohol oder Mischungen davon bestehenden Gruppe extrahiert wird.

5. Gemischter Extrakt von *Atractylodes Rhizome white* und *Taraxacum Herba* zur Verwendung nach Anspruch 4, wobei ein gemischtes Trockengewichtsverhältnis des gemischten Extrakts von *Atractylodes Rhizome white* und *Taraxacum Herba* 1:2-2:1 beträgt.

## Revendications

1. Extrait mixte d'*Atractylodes Rhizome blanc* et de *Taraxacum Herba* pour une utilisation dans la prévention et le traitement d'une maladie intestinale inflammatoire.

2. Extrait mixte d'*Atractylodes Rhizome blanc* et de *Taraxacum Herba* pour une utilisation selon la revendication 1 :
l'*Atractylodes Rhizome blanc* ayant pour origine un ou plusieurs éléments choisis dans un groupe constitué par *Atractylodes japonica, Atractylodes macrocephala* et *Atractylodes ovate* ; et
le *Taraxacum Herba* ayant pour origine un ou plusieurs éléments choisis dans un groupe constitué par *Taraxacum platycarpum, Taraxacum officinale, Taraxacum mongolicum, Taraxacum coreanum, Taraxacum albidum, Taraxacum glabrisquamum, Taraxacum pseudoalbidum* et *Taraxacum taquetii.*

3. Extrait mixte *d'Atractylodes Rhizome blanc* et de *Taraxacum Herba* pour une utilisation selon la revendication 2, *l'Atractylodes Rhizome blanc* ayant pour origine *l'Atractylodes macrocephala.*

4. Extrait mixte *d'Atractylodes Rhizome blanc* et de *Taraxacum Herba* pour une utilisation selon la revendication 3, l'extrait mixte *d'Atractylodes Rhizome blanc* et de *Taraxacum Herba* étant extraits avec un ou plusieurs solvants choisis dans un groupe constitué par l'eau, un alcool en C₁₋₄ ou des mélanges correspondants.

5. Extrait mixte *d'Atractylodes Rhizome blanc* et de *Taraxacum Herba* pour une utilisation selon la revendication 4, un rapport de poids sec mixte de l'extrait mixte d'*Atractylodes Rhizome blanc* et de *Taraxacum Herba* étant de 1:2 à 2:1.
